# EUROPEAN PATENT APPLICATION

(11) **EP 2 374 427 A1**
(43) Date of publication of application: **12.10.2011**
(21) Application number: 10785934.0
(22) Date of filing: 07.06.2010
(51) Int. Cl.: A61B 18/20, A61B 1/00, A61N 5/06

(54) **OPTICAL DEVICE, LASER IRRADIATION DEVICE AND LASER THERAPY DEVICE**

(30) Priority: 09.06.2009 JP 2009138342
(71) Applicant: Fujikura, Ltd., Tokyo 135-8512 (JP)
(72) Inventor: NAKATATE, Kenichi, Sakura-shi Chiba 285-8550 (JP)
(74) Representative: Cabinet Plasseraud
(86) International application number: PCT/JP2010/003777
(87) International publication number: WO 2010/143402

(57) **Abstract**

An optical device that allows laser beams to be incident to one end of an image fiber and receives a two-dimensional image of a laser irradiation target transmitted through the image fiber. The optical device includes: a mirror that is arranged on the one end side of the image fiber, reflects the laser beams, and transmits the two-dimensional image; a laser beam source that allows the laser beams to be incident to the one end of the image fiber through reflection of the mirror; an imaging device that receives the two-dimensional image from the one end of the image fiber through transmission of the mirror; and an incidence control device that allows the laser beams to be incident to some cores out of the plurality of cores in the one end of the image fiber and changes cores to which the laser beams are incident.

## Description

### Technical Field

The present invention relates to an optical device and a laser irradiation device capable of transmitting laser beams and transmitting an image and a laser treatment apparatus using the device.
Priority is claimed on Japanese Patent Application No. 2009-138342, filed on June 9, 2009, the contents of which are incorporated herein by reference.

### Background Art

Laser irradiation devices that transmit laser beams using optical fibers are used for various uses such as medical use, industrial use, and the like.
In a conventional laser treatment system, an optical fiber used for laser beam transmission is configured separately from an optical fiber used for image transmission for performing observation. According to this conventional system, the image of an affected area is checked through the optical fiber used for image transmission, the tip end portion of the optical fiber for laser beam transmission is guided to a position appropriate for irradiation of the laser beams for the affected area based on the image information, and the laser beams can be emitted.
For example, in Patent Document 1, in a surgical device that allows laser beams used for photocoagulation to be incident from one end of an optical fiber and irradiates the affected area with the laser beams so as to photocoagulate the affected area, a configuration in which an illumination light source and an operation light source are integrated as one is disposed.

In the conventional laser treatment system, the accuracy of the position alignment when the front end portion of the optical fiber used for laser beam irradiation is placed toward the affected area largely depends on the function and the determination of an operator, and accordingly, there is a concern that the effects of the laser treatment will be unstable.
Therefore, an endoscopic system having a composite-type optical fiber acquired by combining an optical fiber used for image transmission and an optical fiber used for laser beam transmission is proposed (for example, see Patent Document 2).

### Patent Documents

[Patent Document 1] Japanese Unexamined Patent Application, First Publication No. 2003-111789
[Patent Document 2] Japanese Unexamined Patent Application, First Publication No. 2005-237436

### Disclosure of the Invention

### Problem that the Invention is to solve

However, in the case of an endoscopic system having the above-described composite-type optical fiber, the optical fiber used for laser beam transmission that is not involved in the image transmission is built inside the composite-type optical fiber, and accordingly, a blank portion is formed in the center of an image that is acquired through the optical fiber used for image transmission.
In addition, the position of the optical fiber used for laser beam transmission is fixed in the center of the optical fiber for image transmission, and accordingly, the range in which the laser irradiation can be performed is limited on the center portion of the range in which an image can be observed. When the position or the number of the optical fibers used for laser beam transmission is changed, although the position and the number of points (the number of irradiation spots) of the laser irradiation can be changed within the range in which an image can be observed, it is very complicated to use a different composite-type optical fiber each time the position and the number of the points are changed.

The present invention was made in consideration of the above-described situation, and an object thereof is to provide an optical device and a laser irradiation device capable of easily changing the position and the number of points of laser irradiation within an image observing range and a laser treatment apparatus using the device.

### Means for solving the Problem

In order to achieve the above-described object, according to the present invention, there is provided an optical device, in which, to one end of an image fiber configured by a plurality of cores configuring a pixel and a common clad, laser beams to be output toward a laser irradiation target disposed in the other end of the image fiber are incident, and a two-dimensional image of the laser irradiation target transmitted through the image fiber is received. The optical device includes: a mirror that is arranged on the one end side of the image fiber, reflects the laser beams, and transmits the two-dimensional image; a laser beam source that allows the laser beams to be incident to the one end of the image fiber through reflection of the mirror; an imaging device that receives the two-dimensional image from the one end of the image fiber through transmission of the mirror; and an incidence control device that allows the laser beams to be incident to some cores out of the plurality of cores in the one end of the image fiber and changes cores to which the laser beams are incident.
As the above-described incidence control device, a mask having a transmission portion that transmits the laser beams may be used, and the mask may be arranged within an optical path between the laser beam source and the one end of the image fiber.
The above-described incidence control device may be configured so as to control an irradiation position or a shape of the laser beams by adjusting the angle of the mirror.
It is preferable if the above-described mask is selectable so as to be used from among a plurality of the masks having the transmission portions that have different positions or shapes.
According to the present invention, there is provided a laser irradiation device including: an image fiber that has an image fiber main body configured by a plurality of cores configuring a pixel and a common clad, outputs laser beams, which are incident from one end, from the other end toward a laser irradiation target, and transmits an image signal representing a two-dimensional image of the laser irradiation target from the other end to the one end; a mirror that is arranged on the one end side of the image fiber, reflects the laser beams, and transmits the two-dimensional image; a laser beam source that allows the laser beams to be incident to the one end of the image fiber through reflection of the mirror; an imaging device that receives the two-dimensional image from the one end of the image fiber through transmission of the mirror; an incidence control device that allows the laser beams to be incident to some cores out of the plurality of cores in the one end of the image fiber and changes cores to which the laser beams are incident; and an illumination optical fiber.
As the above-described incidence control device, a mask having a transmission portion that transmits the laser beams may be used, and the mask may be arranged within an optical path between the laser beam source and the one end of the image fiber.
The above-described incidence control device may be configured so as to control an irradiation position or a shape of the laser beams by adjusting an angle of the mirror.
It is preferable if the above-described mask is selectable so as to be used from among a plurality of the masks having transmission portions that have different positions or shapes.
In the laser irradiation device according to the present invention, it may be configured so that the mirror is a wavelength selecting mirror, and the incidence control device is an incidence position control device.
According to the present invention, there is provided a laser treatment apparatus including the above-described laser irradiation device, wherein the image fiber is inserted into a pipe having an outer diameter of 20G or less so as to configure a probe.

### Effects of the Invention

According to the present invention, image transmission together with laser beam transmission can be performed by using one image fiber, and the position and the number of points of laser irradiation can be easily changed within the image observation range.

### Brief Description of the Drawings

FIG. 1 is a schematic diagram illustrating the operation of a laser irradiation device according to the present invention.
FIG. 2 is a cross-sectional view illustrating an example of an image fiber that is used in a laser irradiation device according to the present invention.
FIG. 3 is a schematic diagram illustrating an example of a laser irradiation device according to the present invention.
FIG. 4 is an explanatory diagram illustrating an example in which multipoint irradiation is performed within the observation range of an image.
FIG. 5 is a plan view illustrating an example of a mask.
FIG. 6 is a plan view illustrating an example of a mask.
FIG. 7 is a plan view illustrating an example of a mask.
FIG. 8 is a plan view illustrating an example of a mask.
FIG. 9 is a plan view illustrating an example of a mask.
FIG. 10 is a plan view illustrating an example of a mask.
FIG. 11 is a configuration diagram showing the structure of an emission unit of a laser beam source.
FIG. 12 is a schematic diagram illustrating a laser irradiation device according to another embodiment of the present invention.
FIG. 13 is a schematic diagram illustrating a main portion of a modified example of the laser irradiation device shown in the previous diagram.

### Description of Embodiments

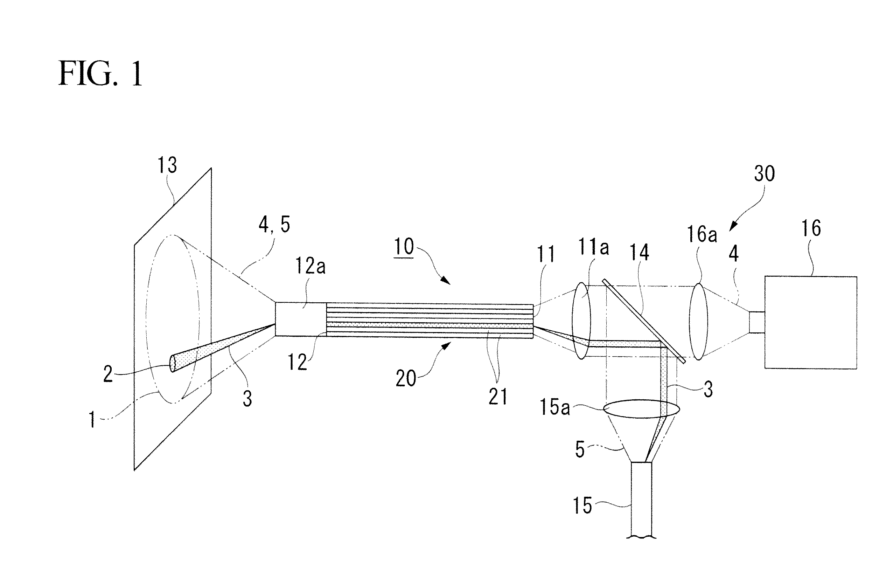

Hereinafter, the present invention will be described based on a preferred embodiment with reference to the drawings.
As illustrated in FIG. 1, the laser irradiation device 10 of this embodiment includes: an image fiber 20 that outputs laser beams 3, which are incident from one end 11, from the other end 12 toward a laser irradiation target 13 and transmits a two-dimensional image of the laser irradiation target 13 from the other end 12 to the one end 11; a mirror 14 that is arranged on the one end 11 side of the image fiber 20, reflects the laser beams 3, and allows the two-dimensional image of the laser irradiation target 13 to pass therethrough; a laser beam source 15 that allows the laser beams 3 to be incident to the one end 11 of the image fiber 20 through the reflection of a mirror 14; an imaging device 16 that receives the two-dimensional image of the laser irradiation target 13 from the one end 11 of the image fiber 20 through the transmission of the mirror 14; and an illumination optical fiber (not shown in the figure) that is used for transmitting illumination light to the laser irradiation target 13.

In this laser irradiation device 10, the laser beams 3 emitted from the laser beam source 15 are allowed to be incident to some cores 21 (one or a plurality of cores) out of a plurality of cores 21, 21, ... in the one end 11 of the image fiber 20 by an incidence control device that is not shown in the figure. The core 21 to which the laser beams 3 are incident can be changed.

The mirror 14, the laser beam source 15, the imaging device 16, the incidence control device, and the illumination optical fiber configure an optical device 30. The optical device 30 allows the laser beams 3 to be incident to the one end 11 of the image fiber 20 and receives the two-dimensional image transmitted through the image fiber 20.

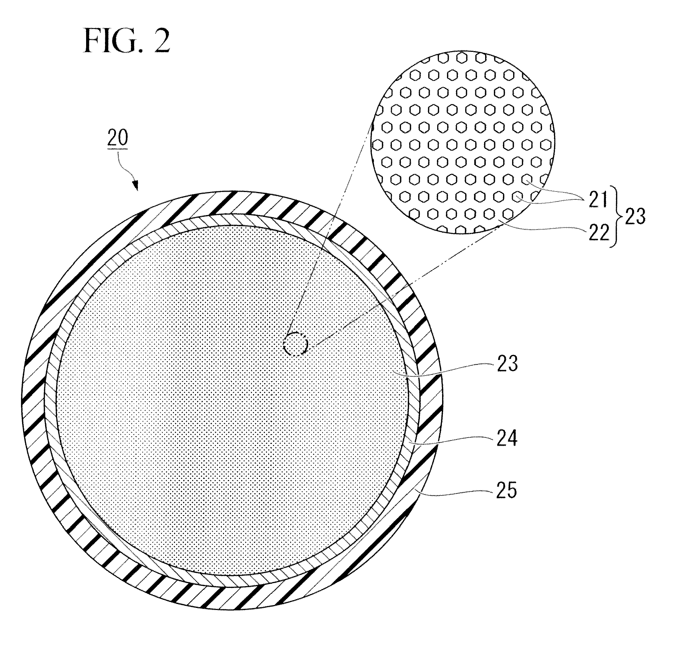

The image fiber 20, as illustrated in FIG. 2, is a multi-core fiber having an image fiber main body 23 that is configured by a plurality of cores 21, 21, ... configuring a pixel and a common clad 22.
In the present invention, the image fiber 20 that can transmit both the wave band of the laser beams emitted to the laser irradiation target 13 and the wave band of light (the light of image information) representing the two-dimensional image of the laser irradiation target 13 is used. The material of the image fiber main body 23 can be selected from silica-based glass, multicomponent glass, plastic, and the like.

In the case of a silica-based image fiber, the core 21 is formed from pure silica glass, silica-based glass in which phosphorus (P), germanium (Ge), or the like is doped (added), or the like.
The clad 22 is made from a material having a refractive index that is lower than that of the core 21 and is formed from pure silica glass, silica-based glass in which fluorine (F) or the like is doped (added), or the like.
Particularly, in the case of a silica-based image fiber having a small diameter and many pixels, it is preferable that the core 21 is formed from silica-based glass to which germanium is added, and the clad 22 is formed from silica-based glass to which fluorine is added.

The core 21 is arranged on the entire cross-section (image circle) of the image fiber main body 23 almost uniformly. Here, "being arranged almost uniformly" means not being biased to a partial area of the cross-section of the image fiber main body 23 but being arranged over the entire area.
The cores 21, 21, ... of the image fiber 20 are almost uniformly arranged within the cross-section of the image fiber main body 23. Accordingly, the laser beams 3 can be transmitted to the laser irradiation target 13 by allowing the laser beams to be incident to any core 21 that is selected from the plurality of cores 21, 21, .... The materials and the dimensions of the cares 21, 21, ... of the image fiber 20 can be formed to be uniform, but are not limited thereto. In other words, the materials or the dimensions of the cores 21 of the image fiber 20 may be individually changed.
The number of the cores 21 to which the laser beams 3 are incident may be one or two or more.
The range in which the cores 21, 21, ... are arranged within the cross-section of the image fiber 20 similarly corresponds to a range 4 in which the two-dimensional image is transmitted and a range 5 in which the laser beams 3 can be transmitted.

Generally, the cross-sectional shape (the shape of the cross-section perpendicular to the longitudinal direction) of the cores 21, 21, ... of the image fiber 20 is an isotropic shape such as a circular shape or a hexagonal shape. Other than that shape, as the cross-sectional shape of the core 21, there are shapes having anisotropy such as an oval shape, an oblong shape, a rectangular shape, or a rhombic shape.
It is preferable that the number of the cores 21 (the number of pixels) is approximately 1000 to 100000. The outer diameters of the cores 21 may be the same but are not limited thereto. In other words, the cores 21 may individually have different outer diameters. The outer diameter of the core 21, for example, may be 1 to 20 µm.
The intervals of the cores 21 that are adjacent to each other (the pixel interval) may be almost constant or may not be constant. The intervals of the cores 21, for example, may be 1.1 to 4 times the outer diameter of the core 21. The intervals of the cores 21 are set based on the refractive index difference between the core 21 and the clad 22. The refractive index difference may be 2 to 5% and is more preferably 3.5 to 4%.

In the case of the image fiber 20 shown in FIG. 2, on the outer circumference of the image fiber main body 23, a jacket tube 24 is disposed, and the outer circumference of the jacket tube 24 is covered with a coating layer 25.
The jacket tube 24 is formed from pure silica glass, and, for example, a material acquired by adding titanium oxide, copper oxide, or the like to silica glass or the like other than that may be used. The jacket tube 24 is used for holding a plurality of optical fibers at the time of manufacturing the image fiber main body 23 by binding and melting a plurality of optical fibers each having a single core so as to be integrated. When such optical fibers are melted so as to be integrated, the clads of the optical fibers are continuous so as to form a common clad 22, and the common clad 22 and the jacket tube 24 are fixed.

The coating layer 25 is formed from a resin such as epoxy acryl series, polyimide, or silicone, metal, or the like. The coating layer 25 may be formed as one layer or a plurality of laminated layers. Preferably, the thickness of the coating layer 25 is approximately 20 to 100 µm.
For the medical purpose of performing laser irradiation inside the body (inside an organ), an opening is formed in a membrane of the skin or the inside of the body, and a probe needs to be inserted therein. This probe has a structure in which an image fiber 20 is inserted into the inside of a pipe (a protection pipe) of a metal or the like and passes through it.
In the case of the laser irradiation device 10 of this embodiment, since the image fiber 20 serves as both an optical fiber for image transmission and an optical fiber for laser beam transmission, the opening used for inserting the probe at the time of irradiation of an affected area inside the body with laser beams can be formed so as to be small.
In addition, in this embodiment, the illumination optical fiber configures another probe in addition to the probe of the laser irradiation device 10 of this embodiment. In this case, the number of openings used for inserting the probe is two, which include the opening for the probe for image transmission and laser beam transmission and the opening for the probe for illumination.

Conventionally, a probe having a probe diameter (the outer diameter of the protection pipe) of 20G (an outer diameter of 0.89 mm), 2 1 G (an outer diameter of 0.81 mm), 23G (an outer diameter of 0.64 mm), 25G (an outer diameter of 0.51 mm), or the like is used. Accordingly, it is preferable that the probe diameter is less than or equal to 20G, and it is more preferable that the probe diameter is less than or equal to 23G.
Since the thickness of the protection pipe is usually greater than or equal to 50 µm, in the case of configuring a probe of 23G, the inner diameter of the pipe is less than or equal to 0.54 mm. Thus, in order to acquire the clearance that is necessary for the insertion, the outer diameter (coating diameter) in the coating layer 25 of the image fiber 20 needs to be formed smaller than the inner diameter of the pipe.
In addition, in the present invention, similarly to Patent Document 2, the illumination optical fiber used for transmitting illumination light to the laser irradiation target may be disposed inside the probe of the laser irradiation device 10 of this embodiment together with the image fiber 20. In this case, the number of openings that are used for inserting the probe is one. As the illumination optical fiber, several optical fibers, which are formed from multicomponent glass or the like and which have an outer diameter of 30 to 70 µm, are housed inside the pipe of the probe.

In the other end 12 of the image fiber 20, an objective lens 12a is disposed. As an example of the configuration of the objective lens 12a, there is a convex lens, a cylindrical lens, or the like that is bonded using an optical (translucent) adhesive agent such as an epoxy-based adhesive. In addition, the end faces of the lens and the image fiber may be directly bonded together or may not be directly bonded together. In other words, by inserting the lens and the image fiber into a sleeve made of metal or the like and respectively bonding the lens and the sleeve and the lens and the image fiber, the positional relationship of the lens and the image fiber can be fixed. Accordingly, the adhesive agent can be prevented from deteriorating by the light transmitted through the image fiber 20 so as to be degraded.
As the material of the convex lens, the cylindrical lens, or the like that configures the objective lens 12a, there is a silica-based glass, multicomponent glass, plastic, or the like. In addition, as this objective lens 12a, a GRIN rod lens can be used. This GRIN rod lens is a cylindrical lens having a refractive index distribution of a graded index type. It is preferable that the end face of the GRIN rod lens is mirror-polished. In a case where a part of the image fiber 20 that is acquired by excluding the coating layer 25 (the image fiber main body 23 and the jacket tube 24) is composed of silica-based glass, the silica-based rod lens may be fusion-spliced so as to be used as the objective lens 12a.

The mirror 14 is disposed on the one end 11 side of the image fiber 20 and is an optical device that reflects the laser beams 3 and allows a two-dimensional image of the laser irradiation target 13 to pass therethrough. As an optical device having such a function, there is a wavelength selecting mirror, that is a device (dichroic mirror) that reflects light having a specific wavelength and allows light having the other wavelengths to pass through it, a device (polarization beam splitter) that separates incident light into polarized components and allows the polarized components to be reflected or pass through it, or the like.
It is preferable that the transmissivity of the mirror 14 at the wavelength of the laser beams 3 and a wavelength adjacent thereto is sufficiently low such that, even in a case where a part of the laser beam 3 is reflected from the one end 11 of the image fiber 20, the reflected light is not incident to the imaging device 16.

In a case where the wavelength of the laser beam 3 is out of the wavelength range of visible light, for example, a near-infrared ray or the like, it is preferable that the mirror 14 allows the entire range of the visible light to pass though it for superior color reproducibility of an image. It is preferable that such a mirror 14 reflects light of a long wavelength side at least including the wavelength band of the laser beams 3 and allows light of a short wavelength side including the wavelength band of visible light to pass through it.
In a case where the wavelength of the laser beams 3 is in the wavelength range of visible light, it is also preferable that the mirror 14 reflects the wavelength of the laser beams 3 and a narrow band near the wavelength for minimizing the change in the color shade of an image.
In addition, in a case where the mirror 14 allows three wavelength bands corresponding to three primary colors, which are appropriately selected, to selectively pass therethrough and reflects other wavelengths, the wavelength of the laser beam 3 can be selected from a broad reflection band.

The laser beam source 15 is arranged at such a position that, as the laser beams 3 emitted from the laser beam source 15 are reflected from the mirror 14, the reflected laser beams 3 are incident to the one end 11 of the image fiber 20.
The type of the laser beam source 15 can be selected depending on the purpose of irradiating the laser irradiation target 13 with the laser beam 3. A laser beam source that emits laser beams having wavelengths of a visible band to a near-infrared band, for example, a dye laser, an argon ion laser, a semiconductor laser, an Nd:YAG laser, a Ho:YAG laser, or the like can be used. In addition, an excimer laser of XeCl, KrF, ArF, or the like can be used. Among them, a laser beam source that emits laser beams as near-infrared light, for example, an Nd:YAG laser (a wavelength of 1.06 µm), or a Ho:YAG laser (a wavelength of 2.1 µm) is preferable. In addition, a green laser such as a double wave (a wavelength of 0.53 µm) of an Nd: YVO (wavelength 532 nm) or Nd:YAG laser or the like is also preferable. In addition, a fiber laser that uses a rare-earth doped fiber as an amplifier and has the entire light path configured by optical fibers may be used. For example, a green light source of 532 nm in which a fiber laser having a wavelength of 1064 nm is used, and the wavelength is converted by using a SHG crystal can be configured. The fiber laser has an advantage of decreasing the optical diameter of emitted light.
The laser beam 3 may be a continuous laser beam or a pulse laser. In addition, On/Off of the irradiation or the irradiation time may be configured to be controlled by disposing a shutter in the laser beam source 15 or in the middle of the optical path of the laser beam 3.

The imaging device 16 is arranged at such a position that a transmitted two-dimensional image can be received by allowing the two-dimensional image of the laser irradiation target 13, which is output from the one end 11 of the image fiber 20, to pass through the mirror 14. This imaging device 16 receives the image transmitted through the image fiber 20 from the one end 11 of the image fiber 20.
As the imaging device 16, there is a CCD camera, an image sensor, or the like. By converting the image received by the imaging device 16 into a signal and transmitting the signal to a display device (not shown in the figure), an operator can visually observe the image. The operator can operate the laser irradiation device 10 based on the observation of the image output to the display device.
As the display device, various types of monitors such as a liquid crystal display device and a CRT can be used.

In addition, the laser irradiation device 10 of this embodiment includes an incidence control device that can change the core 21 to which the laser beam 3 is incident in one end 11 of the image fiber 20, and as illustrated in FIG. 1, accordingly, the irradiation position 2 of the laser beam 3 can be arbitrarily selected in the observation range 1 of the two-dimensional image on the laser irradiation target 13.
Since the position at the one end 11 of the image fiber 20 and the position of the laser irradiation target 13 are conjugates, the image of the laser irradiation target 13 that is acquired by the objective lens 12a is on the end surface in the one end 11 of the image fiber 20. Therefore, by controlling the incidence position of the laser beam 3 in the one end 11 of the image fiber 20, the irradiation position 2 of the laser beam 3 in the laser irradiation target 13 can be controlled. Accordingly, the irradiation position 2 of the laser beam 3 in the observation range 1 of the two-dimensional image can be easily controlled.
The irradiation position 2 of the laser beam 3 can be selected from the center portion or the peripheral portion of the observation range 1 of the two-dimensional image.

In this embodiment, a first collimate lens 11a is disposed between the one end 11 of the image fiber 20 and the mirror 14, a second collimate lens 15a is disposed between the laser beam source 15 and the mirror 14, and a third collimate lens 16a is disposed between the imaging device 16 and the mirror 14.
Although these collimate lenses 11a, 15a, and 16a are not essential configurations, by collecting the laser beams 3 incident to the one end 11 of the image fiber 20 using the first collimate lens 11a, the core 21 to which the laser beams 3 are incident can be easily selected.
In addition, by parallelizing the light 4 of the image information output from the one end 11 of the image fiber 20 using the first collimate lens 11a and then allowing the light to pass through the mirror 14, the disturbance of the image at the time of passing through the mirror 14 can be suppressed.
By parallelizing the laser beams 3 emitted from the laser beam source 15 using the second collimate lens 15a, the disturbance of the shape of the beams at the time when the laser beams 3 are reflected from the mirror 14 can be suppressed.
By collecting the light 4 of the image information that is incident to the imaging device 16 using the third collimate lens 16a, the light can be easily allowed to be incident to the imaging device 16.
As an example of the incidence control device, there is a device that collects the laser beam 3 such that the beam diameter of the laser beam 3 is smaller than the circle diameter (the diameter of the image fiber main body 23) of the image fiber 20 and controls the position of the laser beam incident to the one end 11 of the image fiber 20. To be more specific, a mechanism that controls the position and the direction of emission of the laser beam 3 by moving the laser beam source 15 or controls the position and the angle by moving the lenses 11a and 15a and the mirror 14 or the like can be used.

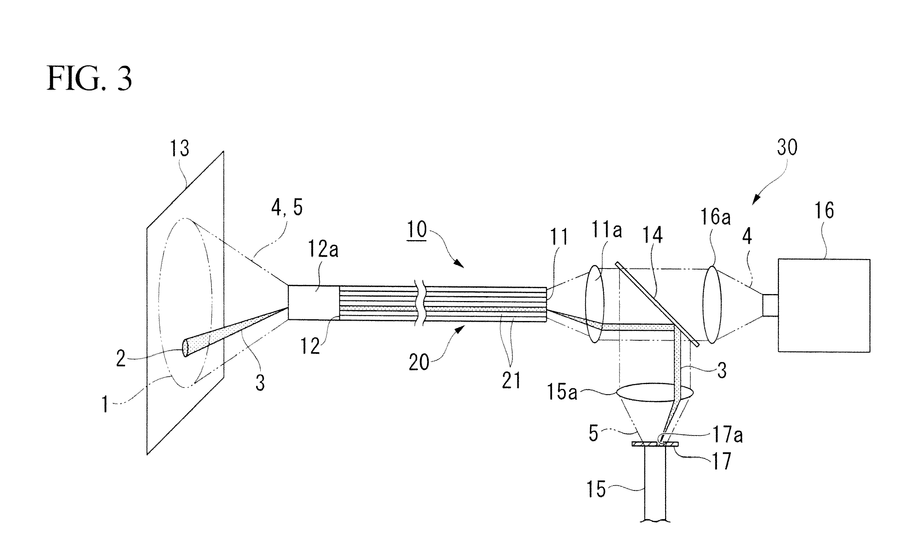

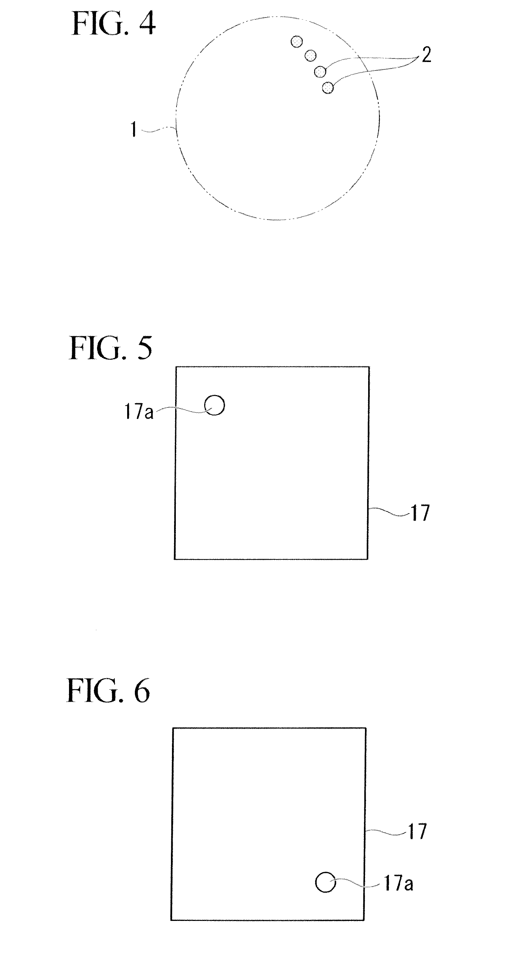

As another example of the incidence control device, as illustrated in FIG. 3, there is a mask 17 that partially has a transmission portion 17a that allows the laser beam 3 to pass therethrough and allows only a part of the cross-section of the laser beam emitted from the laser beam source 15 to pass.
In such a case, by changing the positions or the number of portions of the mask 17 through which the laser beams pass through, for example, as illustrated in FIG. 4, a plurality of spot-shaped irradiation positions 2 is set in the observation range 1 of a two-dimensional image, and the laser irradiation can be simultaneously performed for the irradiation positions 2 in an easy manner.

By preparing a plurality of the masks 17 having different numbers or dispositions of the transmission portions 17a, the mask 17 can be appropriately changed and used.

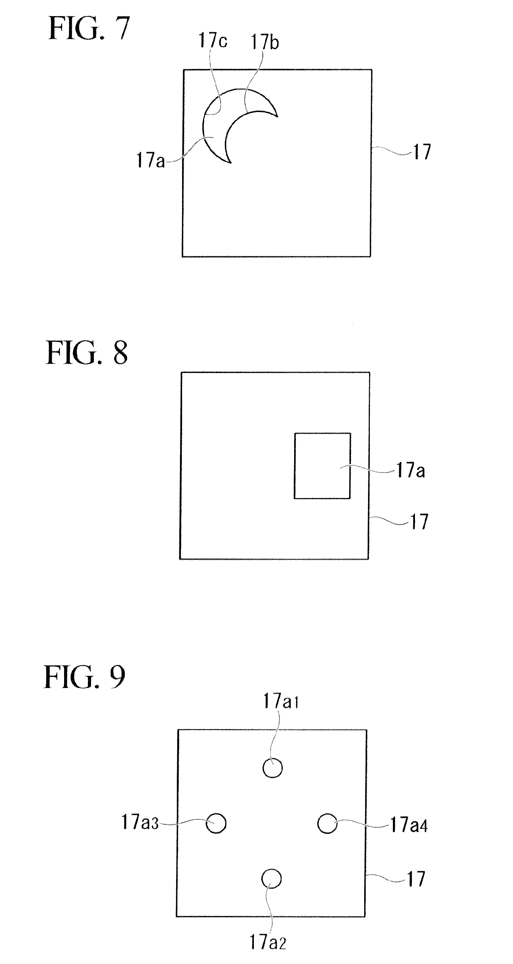

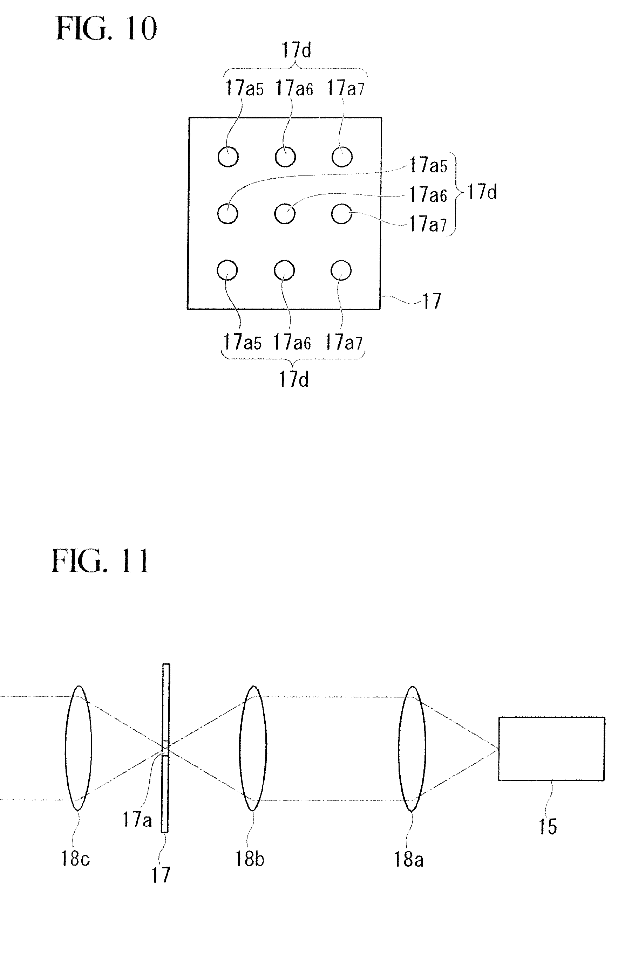

FIGS. 5 to 10 are plan views illustrating examples of the mask 17, and the mask illustrated in FIG. 5 has a rectangular shape, and one circular transmission portion 17a is formed at an upper left position therein. In the mask 17 illustrated in FIG. 6, one circular transmission portion 17a is formed at a lower right position. In the mask 17 illustrated in FIG. 7, a crescent moon-shaped transmission portion 17a of which the inner edge 17b and the outer edge 17c are formed in arc shapes is formed at an upper left position. In the mask 17 illustrated in FIG. 8, one rectangular transmission portion 17a is formed at a position close to the lateral side (right side). In the mask 17 illustrated in FIG. 9, one pair of transmission portions 17a1 and 17a2 that are vertically separated in the center in the widthwise direction (horizontal direction) and one pair of transmission portions 17a3 and 17a4 that are separated in the widthwise direction (horizontal direction) in the center in the vertical direction are formed. In the mask 17 illustrated in FIG. 10, multiple rows (three rows in the example illustrated in the figure) of columns 17d formed from a plurality of (three in the example illustrated in the figure) transmission portions 17a (17a5 to 17a7), which are linearly arranged along the horizontal direction, are formed with a gap interposed therebetween in the vertical direction.
As illustrated in FIGS. 5 to 10, as the masks 17, a plurality of masks having different positions and shapes of the transmission portions 17a is prepared and can be selected so as to be used as needed.

In addition, by changing the relative position of the transmission portion 17a within the range 5 in which the laser beam 3 can be transmitted by moving the mask 17 in a direction perpendicular to the traveling direction of the laser beam 3 in the optical path of the laser beam 3, the irradiation position 2 of the laser beam 3 in the observation range 1 of a two-dimensional image can be controlled.

The mask 17 can be arranged at any position, as long as the position is a position on an optical path of the laser beam 3 not interfering with other optical devices. In order not to increase the diameter of the probe when the probe including the image fiber 20 is inserted into the inside of the body or the like, it is preferable that the mask is arranged on the one end 11 side of the image fiber 20.
As illustrated in FIG. 3, in a case where the mask 17 is arranged on an end surface (in contact with or in proximity thereto) of the emission of the laser beam source 15, it is difficult for the positional relationship between the mask 17 and the laser beam source 15 to be misaligned, which is preferable.
Described in detail, a configuration may be employed in which a collimate lens (not shown in the figure) is disposed between the end surface of the emission of the laser beam source 15 and the mask 17, the emitted light is parallelized by the lens, and a part of the parallelized light is allowed to pass through the transmission portion 17a of the mask 17.
In addition, as illustrated in FIG. 11, a configuration may be employed in which collimate lenses 18a and 18b are disposed between the laser beam source 15 and the mask 17, a collimate lens 18c is disposed on the output side of the mask 17, the emitted beams are parallelized by the collimate lens 18a, are collected by the collimate lens 18b, are allowed to pass through the transmission portion 17a of the mask 17 at the light collecting position, and are parallelized again by the collimate lens 18c.

In addition, in a case where the collimate lenses 11a and 15a are arranged on the optical path of the laser beams 3, when the mask 17 is disposed within a range in which the laser beams 3 are parallelized between the collimate lenses 11a and 15a, even in a case where there is an error in the position of the mask 17 along the traveling direction of the laser beams 3 on the optical path of the laser beams 3, it is difficult for the laser irradiation position 2 to be misaligned, which is preferable.

As described above, according to the laser irradiation device 10 of this embodiment, the laser irradiation target 13 can be observed based on the image transmitted through the image fiber 20. In addition, the laser irradiation target 13 can be irradiated with the laser beams 3 through the image fiber 20.
In the image fiber 20, since the cores 21 are arranged almost uniformly on the entire cross section of the image fiber main body 23, a blank portion is not generated in an acquired image, and there is no limitation on the irradiation position of the laser beams 3. Accordingly, the laser beams 3 can be assuredly emitted to necessary positions within the observation range 1 of the image.
Accordingly, for example, an affected area can be discovered and diagnosed based on the image acquired through the image fiber 20, and a laser treatment can be performed by irradiating the affected area with the laser beams.
At this time, there is no blank portion in the image, and there is no limitation on the irradiation position of the laser beam 3, and accordingly, the laser beams can be emitted to necessary positions, thereby improving the effects of the treatment.

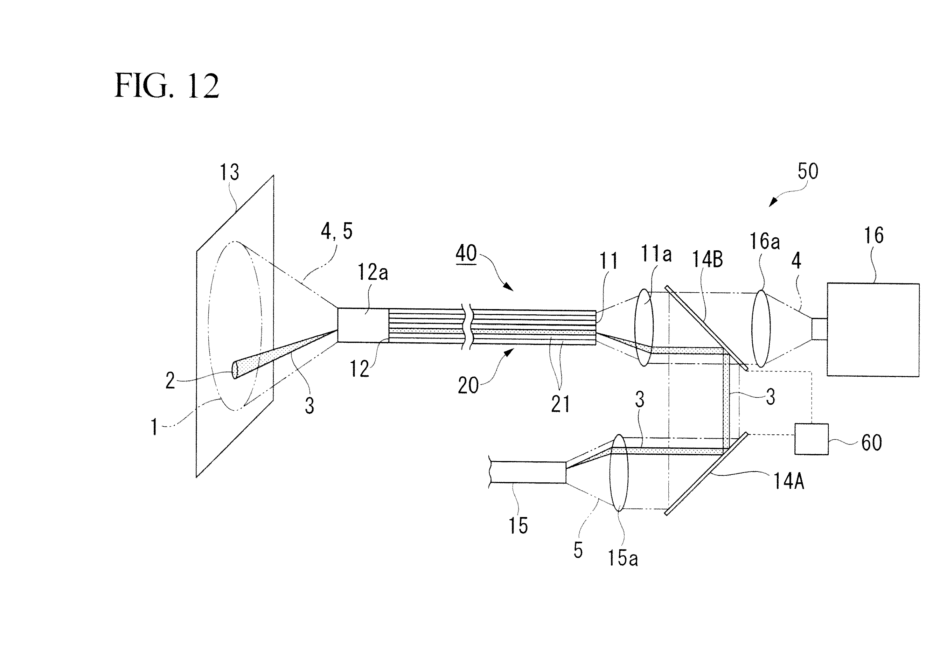

FIG. 12 illustrates another embodiment of the present invention, and, in a laser irradiation device 40 of this embodiment, first and second mirrors 14A and 14B are disposed between an image fiber 20 and a laser beam source 15, and an incidence control device 60 that adjusts the angles of the mirrors 14A and 14B is disposed in the mirrors 14A and 148. As the mirrors 14A and 14B, the same configuration as that of the mirror 14 of the above-described laser irradiation device 10 can be employed. The other configurations are the same as those of the laser irradiation device 10.
In the description presented hereinafter, the same configurations as those of the laser irradiation device 10 illustrated in FIG. 1 are omitted or appropriately simplified.

The mirrors 14A and 14B, the laser beam source 15, an imaging device 16, an incidence control device 60, and an illumination optical fiber configure an optical device 50. The optical device 50 allows laser beams 3 to be incident to one end 11 of the image fiber 20 and receives a two-dimensional image that is transmitted through the image fiber 20.

The incidence control device 60 is configured such that, by rotating first and second mirrors 14A and 14B around different axes using a driving device not shown in the figure, the angles thereof can be arbitrarily set. For example, a configuration may be employed in which the first mirror 14A can rotate around the X axis, and the second mirror 14B can rotate around the Y axis perpendicular to the X axis.
In such a configuration, by adjusting the angles of the mirrors 14A and 14B, the irradiation position of the laser beams 3 for one end 11 of the image fiber 20 can be controlled. Since the rotation axes of the mirrors 14A and 14B are different from each other, the irradiation position of the laser beam 3 can be arbitrarily set.
For example, as illustrated in FIG. 4, in a case where a plurality of spot-shaped irradiation positions 2 is set in the observation range 1 of a two-dimensional image, while the angles of the mirrors 14A and 14B are sequentially adjusted, the laser irradiation can be sequentially performed for the irradiation positions 2.
In addition, although the laser irradiation device 40 illustrated in FIG. 12 has a configuration having two mirrors, a configuration having only one mirror can be employed. In such a case, the incidence control device is configured so as to rotate the one mirror around any of the X axis and the Y axis to be set at an arbitrary angle.

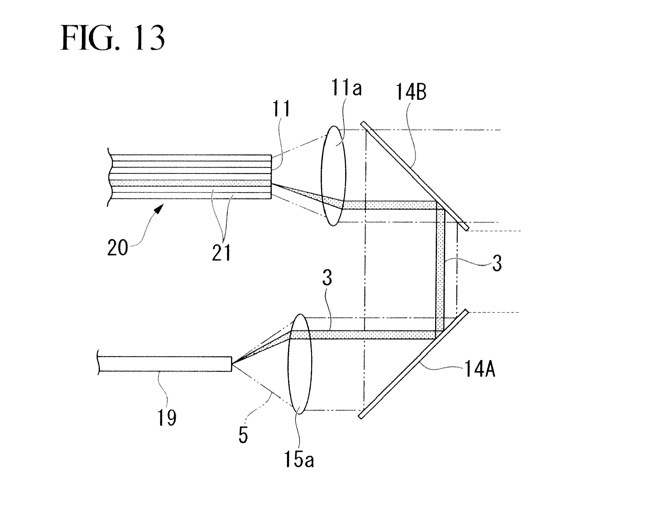

As illustrated in FIG. 13, in the laser irradiation device 40 (see FIG. 12), light emitted from the laser beam source 15 can be output through an optical fiber 19. By using the optical fiber 19, the optical diameter in the one end 11 of the image fiber 20 can be decreased.

A laser treatment apparatus of the present invention can be used so as to accurately eliminate tumor tissue that is located on a boundary between normal tissue and the tumor tissue in the treatment of a brain tumor in brain surgery.
Conventionally, it is very difficult to distinguish a boundary between tumor tissue and the normal tissue of a cerebral nerve, and it is necessary to perform an operation while an affected area is observed. At that time, in a case where a cut end is excessively large, there is a risk of damaging the brain, and, in a case where a large excision is made so that the tumor does not remain, the function of the brain may be damaged. Accordingly, although an endoscopic surgery and a laser treatment that accurately perform an operation with minimal invasion are required, an endoscope and a laser treatment probe are separately used in the current stage, and thereby it is difficult to perform an accurate operation.
In contrast to this, the laser treatment apparatus of the present invention has both the function of an endoscope and the function of a laser treatment probe, and accordingly, a treatment through a partial laser irradiation according to an affected area can be performed, whereby an effective treatment can be made, and the burden on a patient is reduced.

### Industrial Applicability

The laser irradiation device of the present invention can be used for various uses including medical uses, industrial uses, and the like. As the uses for a medical treatment, there are angiogenesis, angiorrhaphy, calculus fragmentation, and the like. To be more specific, the laser irradiation device is appropriate for a laser treatment apparatus for a brain tumor in a cerebral surgery and cancer or a malignant tumor of various portions.
As the industrial uses, there are an operation in an atomic facility or a thin pipe arrangement and the like.

### Description of Reference Numerals and Signs

1: observation range of two-dimensional image
2: irradiation position of laser beams
3: laser beams
10: laser irradiation device
11: one end of image fiber
12: the other end of image fiber
13: laser irradiation target
20: image fiber
14, 14A, and 14B: mirror
15: laser beam source
16: imaging device
17: mask (incidence control device)
17a: transmission portion
21: core
22: common clad
23: image fiber main body
30 and 50: optical device
60: incidence control device

## Claims

1. An optical device in which, to one end of an image fiber configured by a plurality of cores configuring a pixel and a common clad, laser beams to be output toward a laser irradiation target disposed in the other end of the image fiber are incident, and a two-dimensional image of the laser irradiation target transmitted through the image fiber is received, the optical device comprising:
a mirror that is arranged on the one end side of the image fiber, reflects the laser beams, and transmits the two-dimensional image;
a laser beam source that allows the laser beams to be incident to the one end of the image fiber through reflection of the mirror;
an imaging device that receives the two-dimensional image from the one end of the image fiber through transmission of the mirror; and
an incidence control device that allows the laser beams to be incident to some cores out of the plurality of cores in the one end of the image fiber and changes cores to which the laser beams are incident.

2. The optical device according to claim 1,
wherein the incidence control device is a mask having a transmission portion that transmits the laser beams, and
wherein the mask is arranged within an optical path between the laser beam source and the one end of the image fiber.

3. The optical device according to claim 1, wherein the incidence control device controls an irradiation position or a shape of the laser beams by adjusting an angle of the mirror.

4. The optical device according to claim 2, wherein the mask is selectable so as to be used from among a plurality of the masks having the transmission portions that have different positions or shapes.

5. A laser irradiation device comprising:
an image fiber that has an image fiber main body configured by a plurality of cores configuring a pixel and a common clad, outputs laser beams, which are incident from one end, from the other end toward a laser irradiation target, and transmits an image signal representing a two-dimensional image of the laser irradiation target from the other end to the one end;
a mirror that is arranged on the one end side of the image fiber, reflects the laser beams, and transmits the two-dimensional image;
a laser beam source that allows the laser beams to be incident to the one end of the image fiber through reflection of the mirror;
an imaging device that receives the two-dimensional image from the one end of the image fiber through the transmission of the mirror;
an incidence control device that allows the laser beams to be incident to some cores out of the plurality of cores in the one end of the image fiber and changes cores to which the laser beams are incident; and
an illumination optical fiber.

6. The laser irradiation device according to claim 5,
wherein the incidence control device is a mask having a transmission portion that transmits the laser beams, and
wherein the mask is arranged within an optical path between the laser beam source and the one end of the image fiber.

7. The laser irradiation device according to claim 5, wherein the incidence control device controls an irradiation position or a shape of the laser beams by adjusting an angle of the mirror.

8. The laser irradiation device according to claim 6, wherein the mask is selectable so as to be used from among a plurality of the masks having transmission portions that have different positions or shapes.

9. The laser irradiation device according to claim 5,
wherein the mirror is a wavelength selecting mirror, and
wherein the incidence control device is an incidence position control device.

10. A laser treatment apparatus comprising the laser irradiation device according to claim 1,
wherein the image fiber is inserted into a pipe having an outer diameter of 20G or less so as to configure a probe.
